# DEMANDE DE BREVET EUROPEEN

(11) **EP 1 252 883 A1**
(43) Date de publication de la demande: **30.10.2002**
(21) Numéro de dépôt: 02291031.9
(22) Date de dépôt: 23.04.2002
(51) Int. Cl.: A61K 7/48, A61K 7/06

(54) **Procédé pour diminuer partiellement ou totalement les symptômes associés à la libération d'histamine dans l'organisme**

(30) Priorité: 23.04.2001 FR 0105458
(71) Demandeur: L'OREAL, 75008 Paris (FR)
(72) Inventeur: De Lacharriere, Olivier, 75015 Paris (FR); Jourdain, Roland, 92360 Meudon La Forêt (FR)
(74) Mandataire: Catherine, Alain

(57) **Abrégé**

Utilisation pour diminuer partiellement ou totalement les symptômes associés à la libération d'histamine dans l'organisme, notamment le prurit et l'érythème, qui comprend l'application topique d'une composition cosmétique et/ou dermatologique et/ou de l'hygiène contenant une quantité effective d'au moins un agent chélateur d'ions, ladite composition étant essentiellement exempte de flavones, de flavonones et/ou de flavonoides et d'agents sequestrants tels que l'acide polyaspartique et ses sels, les dérivés de cellulose et leurs sels, et les copolymères à motifs monomères d'acide maléique ou hydrosuccinique.

## Description

La présente invention se rapporte à un procédé pour diminuer partiellement ou totalement les symptômes associés à la libération d'histamine, notamment l'érythème et le prurit.

L'histamine est une amine dérivée de l'histidine, présente dans les tissus animaux. L'histamine est un médiateur chimique de phénomènes tels que les sécrétions gastriques, les allergies, etc.

Par médiateur chimique, on entend une substance libérée par une cellule et intervenant dans un processus de l'organisme (conduction nerveuse, inflammation). L'histamine est libérée dans l'organisme à partir des mastocytes qui sont des cellules du tissu conjonctif qui sécrètent des substances chimiques participant aux réactions immunitaires et à la coagulation du sang, et qui sont impliquées dans les phénomènes d'allergie.

Les causes de la libération d'histamine à partir des mastocytes sont variées. II existe des causes immunologiques (hypersensibilité réaginique, activation du complément C3a, IgG antirécepteur à IgE de haute affinité Fc_{ε}Rla) et des causes non immunologiques (médicaments, grattages).

Les conséquences de la libération d'histamine sont l'oedème, l'érythème et le prurit (triade de Lewis).

La demanderesse a découvert que l'application topique d'une composition contenant un chélateur d'ions (EDTA) permettait de diminuer les symptômes engendrés par une application d'histamine par iontophorèse, et en particulier le prurit et l'érythème. Ce test est un bon modèle d'évaluation du pouvoir antiprurigineux et anti-érythémateux d'une composition topique.

Le brevet japonais JP-9323920 décrit une composition détergente contenant un sulfate d'ester comme surfactant, un cosurfactant, un bactéricide cationique et un agent chélateur utilisé pour le nettoyage de la peau et des cheveux montrant une activité antibactérienne, antipelliculaire et anti-prurigineuse.

Cependant, ce document ne concerne pas un procédé pour diminuer partiellement ou totalement les symptômes associés à la libération d'histamine. De plus, rien n'est dit concernant l'activité anti-histaminique de l'agent chélateur.

La présente invention a donc l'utilisation pour diminuer partiellement ou totalement les symptômes associés à la libération d'histamine, en particulier l'érythème et le prurit, comprenant l'application topique d'une composition cosmétique et/ou dermatologique et/ou de l'hygiène contenant une quantité effective d'au moins un agent chélateur d'ions, ladite composition étant essentiellement exempte de flavones, de flavonones et/ou de flavonoides et d'agents séquestrants tels que l'acide polyaspartique et ses sels, les dérivés de cellulose et leurs sels, et les copolymères à motifs monomères d'acide maléique ou hydrosuccinique.

Par chélateur d'ions, on entend les composés chimiques ou biologiques (protéines, peptides ...) ayant le pouvoir de séquestrer des ions (anions ou cations).

A titre d'exemple de chélateur chimique, on peut citer:
- l'acide aminotriméthyle phosphonique,
- l'acide β-alanine diacétique,
- l'acide citrique,
- la cyclodextrine,
- l'acide cyclohexanediamine tétracétique,
- l'acide diéthylènetriamine pentaméthylène phosphonique,
- l'acide diéthanolamine N-acétique (diéthanolamine N-acétique),
- l'acide éthylène diamine tétracétique (EDTA ou YH₄) et ses sels de sodium (YH₃Na, Y₂H₂Na₂, YH₃Na₃ et YNa₄), de potassium (YH₃K, Y₂H₃K₃ et YK₄), de calcium disodium, de diammonium et ses sels de triéthanolamine (TEA-EDTA),
- l'acide étidronique,
- l'acide galactanique,
- l'acide hydroxyéthyl éthylènediamine tétracétique (HEDTA) et son sel trisodique,
- l'acide L-tartarique,
- l'acide gluconique,
- l'acide glucuronique,
- l'acide nitrilotriacétique (NTA) et son sel trisodique,
- l'acide pentétique,
- l'acide phytique,
- l'acide ribonique,
- le citrate de diammonium,
- l'azacycloheptane diphosphonate de disodium,
- le pyrophoshate de disodium,
- l'hydroxypropyl cyclodextrine,
- le méthyl cyclodextrine,
- le triphosphate de pentapotassium,
- l'aminotriméthylène phosphonate de pentasodium,
- l'éthylènediamine tétraméthylène phosphonate de pentasodium,
- le pentétate de pentasodium,
- le triphosphate de pentasodium,
- le citrate de potassium,
- EDTMP de potassium,
- EDTMP de sodium,
- le chitosan méthylène phosphonate de sodium,
- l'hexamétaphosphate de sodium,
- le métaphosphate de sodium,
- le polyphosphate de potassium,
- le polyphosphate de sodium,
- le trimétaphosphate de sodium,
- le dihydroxyéthylglycinate de sodium,
- le gluconate de potassium,
- le gluconate de sodium,
- le glucopeptate de sodium,
- le glycereth-1 polyphosphate de sodium,
- le pyrophosphate de tétrapotassium,
- le polyphosphate de triéthanolamine (TEA),
- le pyrophosphate de tétrasodium,
- le phosphate de trisodium,
- l'oxyde triphosphonométhylamine de potassium,
- le métasilicate de sodium,
- le phytate de sodium,
- le polydiméthylglycinophénolsulfonate de sodium,
- le tétrahydroxyéthyl éthylène diamine,
- le tétrahydroxypropyl éthylène diamine,
- l'étidronate de tétrapotassium,
- l'étidronate de tétrasodium,
- l'iminodisuccinate de tétrasodium,
- l'éthylènediamine disuccinate de trisodium,
- l'acide éthanolamine N,N-diacétique,
- l'acétate de disodium,
- le dimercaprol,
- la déferoxamine,
- le Zylox, chélateur du fer décrit et revendiqué dans la demande internationale WO 94/61338,
- sans que cette liste soit limitative.

Un agent chélateur chimique préféré selon l'invention sera choisi parmi l'acide éthylènediamine tétracétique (EDTA) et ses sels de sodium, de potassium, de calcium disodium, de diammonium, de triéthanolamine (TEA-EDTA), l'acide hydroxyéthyl éthylènediamine tétracétique (HEDTA) et son sel trisodique, ainsi que leurs mélanges.

A titre d'exemples de chélateurs biologiques, on peut citer la métallothionéine, la transférine, la calmoduline, le chitosan et ses dérivés, sans que cette liste soit limitative.

L'agent chélateur est présent dans la composition utilisée dans le procédé selon l'invention en une quantité représentant de 10⁻⁶ à 10% en poids, et de préférence de 0,01 à 5% en poids du poids total de la composition.

La composition utilisée dans le procédé selon l'invention peut en outre comporter au moins une phase grasse, liquide ou solide.

Par phase grasse liquide, au sens de la demande, on entend une phase grasse liquide à la température ambiante (25°C) composée d'un ou plusieurs corps gras liquides à température ambiante, également appelés huiles, compatibles entre eux.

Les huiles de la phase grasse des compositions de l'invention peuvent être des huiles, polaires ou non polaires, volatiles ou non volatiles, choisies parmi les huiles classiquement utilisées en cosmétique.

Parmi les huiles polaires, on peut citer les huiles hydrocarbonées comportant des fonctions esters, éthers, acides, alcools ou leurs mélanges, telles que par exemple:
- les huiles hydrocarbonées à forte teneur en triglycérides constitués d'ester d'acides gras et de glycérol, dont les acides gras peuvent avoir des longueurs de chaînes variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raison ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol 810, 812 et 818 par la société Dynamit Nobel,
- les huiles de synthèse de formule R¹COOR² dans laquelle R¹ représente le reste d'un acide gras supérieur, linéaire ou ramifié, comportant de 7 à 19 atomes de carbone, et R² représente une chaîne hydrocarbonée ramifiée contenant de 3 à 20 atomes de carbone, comme par exemple l'huile de Purcellin (octanoate de cétostéaryle), l'isononanoate d'isononyle, les benzoates d'alkyle,
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de diisostéaryle, et les esters du pentaérythritol;
- les alcools gras en C₈ à C₂₆ comme l'alcool oléique; et
- leurs mélanges.

Parmi les huiles apolaires, on peut citer:
- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthiocones, les phényl diméthicones, les phényl triméthylsiloxy diphénylsiloxanes, les diphényldiméthicones, les diphényl méthyldiphényl trisiloxanes, les 2-phényléthyl triméthylsiloxysilicates,
- les hydrocarbures ou les hydrocarbures fluorés, linéaires ou ramifiés d'origine synthétique ou minérale, comme les huiles de paraffine (par exemple les isoparaffines), et les hydrocarbures aliphatiques (par exemple l'isododécane), et leurs dérivés, la vaseline, les polydécènes, le polyisobutène hydrogéné tel que le parléam, le squalane, et leurs mélanges.

De préférence, les huiles de la phase grasse sont des huiles apolaires du type hydrocarboné d'origine minérale ou synthétique, choisies en particulier parmi les hydrocarbures, notamment les alcanes, comme l'huile de parléam, les isoparaffines dont l'isododécane, le squalane et leurs mélanges.

Par phase grasse solide au sens de la demande, on entend un composé gras lipophile, solide à température ambiante (25°C), ayant une température de fusion supérieure à 40°C et pouvant aller jusqu'à 200°C, en d'autres termes une cire.

Les cires sont celles généralement utilisées dans les domaines cosmétique ou dermatologique. Elles sont notamment d'origine naturelle comme la cire d'abeilles, la cire de Carnauba, de Candellila, d'Ouricoury, du Japon, de fibres de liège ou de canne à sucre, les cires de paraffines, de lignite, les cires microscristallines, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par la synthèse de Fischer-Tropsch, les esters d'acides gras et les glycérides concrets à 40°C, les cires de silicone comme les alkyles, alcoxy et/ou esters de poly(di)méthylsiloxane solide à 40°C.

De façon connue, la composition cosmétique utilisée dans le procédé selon l'invention peut également contenir des adjuvants classiquement utilisés en cosmétique tels que l'eau éventuellement épaissie ou gélifiée par un épaississant ou par un gélifiant de phase aqueuse, les antioxydants, les huiles essentielles, les conservateurs, les neutralisants, les polymères liposolubles, les charges, les parfums, les émulsionnants, les gélifiants, les fibres, les absorbeurs d'odeur et les matières colorantes.

Les quantités de ces différents adjuvants sont celles classiquement utilisées en cosmétique, par exemple de 0,01% à 10% du poids total de la composition. Ces adjuvants, selon leur nature, peuvent être introduits dans la phase grasse, dans la phase aqueuse et/ou dans les sphérules lipidiques.

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables selon l'invention, on peut citer les alcools inférieurs, notamment l'éthanol, l'isopropanol et le propylène glycol.

Comme gélifiants hydrophiles selon l'invention, on peut citer les polymères carboxyvinyliques (carbomer), les copolymères acryliques tels que les copolymères d'acrylates/alkylacrylates, les polyacrylamides, les polysaccharides tels que l'hydroxypropyl-cellulose, les gommes naturelles et les argiles.

Comme gélifiants lipophiles, on peut citer les argiles modifiées comme les bentones, les sels métalliques d'acides gras comme les stéarates d'aluminium et la silice hydrophobe, éthylcellulose, polyéthylène.

La composition utilisée dans le procédé selon l'invention peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

La composition utilisée dans le procédé selon l'invention peut également associer au moins un agent chélateur d'ions métalliques à d'autres agents actifs destinés notamment à la rétention et/ou au traitement des affections cutanées. Parmi ces agents actifs, on peut citer à titre d'exemple:
- les agents modifiant la différentiation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone,
- les antibactériens tels que le phosphate de clindamycine, l'érythromycine ou les antibiotiques de la classe des tétracyclines;
- les antiparasitaires, en particulier le métronidazole, le crotamiton ou les pyréthrinoïdes;
- les antifongiques, en particulier les composés appartenant à la classe des imidazoles tels que l'éconazole, le kétoconazole ou le miconazole ou leurs sels,
- les composés polyènes, tels que l'amphotéricine B, les composés de la famille des allylamines, tels que la terbinafine, ou encore l'octopiroxy;
- les agents antiviraux tels que l'acyclovir;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique;
- les agents anesthésiques tels que le chlorhydrate de lidocaïne et ses dérivés;
- les agents antiprurigineux comme la thénaldine, la triméprazine ou la cyproheptadine;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicyclique;
- les agents anti-radicaux libres, tels que l'alpha-tocophérol ou ses esters, les superoxydes dismutases, certains chélateurs de métaux ou l'acide ascorbique et ses esters;
- les anti-séborrhéiques tels que la prosgestérone;
- les antipelliculaires comme l'octopirox ou la pyrithione de zinc;
- les anti-acnéiques comme l'acide rétinoïque ou le peroxyde de benzoyle.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges Cette matière colorante est généralement présente à raison de 0,01 à 40% du poids total de la composition, de préférence de 5 à 25%.

Les colorants liposolubles sont, par exemple, le rouge Soudan, le DC Red 17, le DC Green 6, le carotène, l'huile de soja, le brun Soudan, le DC Yellow 11, le DC Violet 2, le DC orange 5, le jaune quinoléine. Ils peuvent représenter de 0 à 20% du poids de la composition et mieux de 0,1 à 6%.

Les pigments peuvent être blancs ou colorés, minéraux et/ou organiques, enrobés ou non.

On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Les pigments minéraux préférés sont les oxydes de fer, notamment l'oxyde de fer rouge, l'oxyde de fer jaune, l'oxyde de fer rouge et jaune, l'oxyde de fer brun, l'oxyde de fer noir et le dioxyde de titane.

Parmi les pigments organiques, on peut citer:
- le noir de carbone,
- les pigments de type D&C, tel que le D&C Red No 36, et
- les laques à base de carmin de cochenille, de baryum, de strontium, de calcium telle que le D&C Red No 7 calcium lake, d'aluminium, telles que le D&C Red No 27 aluminium lake, le D&C Red No 21 aluminium lake, le FD&C Yellow No 5 aluminium lake, le FD&C Yellow No 6 aluminium lake, le D&C Red No 7 et le FD&D Blue No 1.

Les pigments peuvent représenter de 0 à 40% du poids total de la composition, et de préférence de 2 à 25%.

Les pigments nacrés peuvent être choisis parmi les pigments nacrés blancs, tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité, ainsi que les pigments nacrés à base d'oxychlorure de bismuth. Ils peuvent représenter de 0 à 20% du poids total de la composition et mieux de 0,1 à 15%.

Selon le mode d'administration, la composition utilisée dans le procédé selon l'invention peut se présenter sous toutes les formes galéniques normalement utilisées pour une application topique, destinées particulièrement aux domaines cosmétique et/ou dermatologique et/ou de l'hygiène.

La composition utilisée dans le procédé selon l'invention peut être appliquée sur la peau (sur toute zone cutanée du corps), sur le cuir chevelu ou sur les muqueuses (buccale, jugale, gingivale et conjonctive).

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ioniques. Ces compositions sont préparées selon les méthodes usuelles.

La composition utilisée dans le procédé selon l'invention peut être également utilisée pour le cuir chevelu sous forme de solutions aqueuses, alcooliques ou hydroalcooliques, ou sous forme de crèmes, de gels, d'émulsions, de mousses ou encore sous forme de compositions pour aérosols comprenant également un agent propulseur sous pression.

Les quantités des différents constituants dans les compositions utilisées dans le procédé selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes, comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions utilisées dans le procédé selon l'invention peuvent également consister en des préparations solides constituant des savons ou des pains de nettoyage.

Les compositions peuvent aussi être conditionnées sous forme de composition pour aérosol comprenant également un agent propulseur sous pression.

Lorsque la composition utilisée dans le procédé selon l'invention est une émulsion, la proportion de la phase grasse peut aller de 5% à 80% en poids, et de préférence de 5% à 50% en poids par rapport au poids total de la composition.

L'émulsionnant et le coémulsionnant sont présents, dans la composition, en une proportion allant de 0,3% à 30% en poids, et de préférence de 0,5 0 20% en poids par rapport au poids total de la composition. L'émulsion peut, en outre, contenir des vésicules lipidiques.

Lorsque la composition utilisée dans le procédé selon l'invention est une solution ou un gel huileux, la phase grasse peut représenter plus de 90% du poids total de la composition.

Les procédés selon l'invention peuvent être mis en oeuvre en appliquant les compositions telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions, les proportions indiquées sauf indication contraire sont des pourcentages en poids.

### Exemple

### Test fonctionnel in vivo sur l'effet de l'EDTA vis-à-vis de l'érythème et du prurit induits par application d'histamine par iontophorèse

Un test fonctionnel *in vivo* chez l'homme est réalisé pour mettre en évidence, sur une population à peau normale, les propriétés propres à l'EDTA vis-à-vis des symptômes induits par l'application d'histamine par iontophorèse sur les avant-bras.

### Sujets

12 volontaires sains, de sexe féminin, âges de 18 à 45 ans de phototype I à IV,

### Produits testés

agent chélateur: sel de disodium éthylènediamine tétraacétate (EDTA),
compositions cosmétiques selon l'invention CM₁, CM₂ et CM₃ contenant respectivement 0,05%, 0,5% et 2% d'EDTA et se présentant sous forme de gel.
composition cosmétique CM₀ ne contenant pas d'EDTA (véhicule de CM₁, CM₂ et CM₃) et se présentant sous forme de gel.

Les compositions CM₀ à CM₃ sont présentées dans le tableau 1.

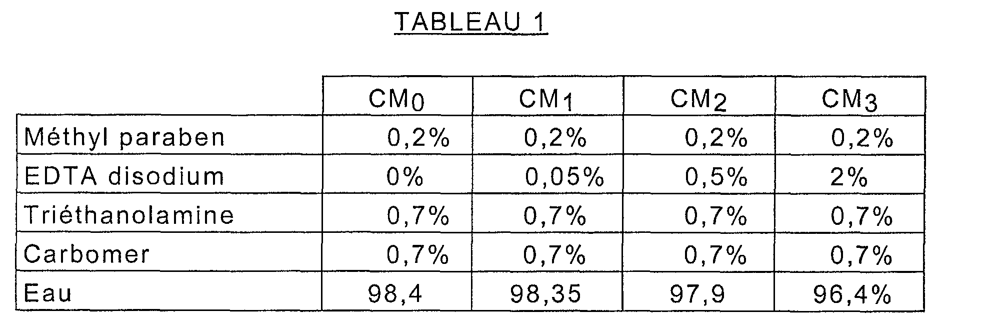

Les compositions CM₁, CM₂ et CM₃ sont testées en monoapplications versus le véhicule (composition CM₀) au cours de 3 visites situées à 15 jours d'intervalle minimum l'une de l'autre
visite 1 : test de la composition CM₁,
visite 2 : test de la composition CM₂,
visite 3 : test de la composition CM₃.

### Méthodologie

II s'agit d'une étude prospective, monocentrique, en double-aveugle, randomisée, contrôlée versus véhicule (composition CM₀) avec une comparaison intra-individuelle (avant-bras droit/avant-bras gauche).

On applique à t = 0 la composition cosmétique CM₀ sur l'un des avant-bras et l'une des compositions cosmétiques CM₁, CM₂ ou CM₃ sur l'autre avant bras.

On applique à t = 30 minutes l'histamine par iontophorèse sur les zones prétraitées.

On évalue ensuite, entre t = 35 minutes et t = 50 minutes: la surface (en cm²) de l'érythème induit par l'histamine à t = 35, 40, 45, 50 minutes
le prurit induit par l'histamine aux mêmes instants c'est-à-dire à t = 35, 40, 45, 50 minutes, en utilisant l'échelle suivante:
0 = pas de sensation
1 = léger ou douteux
2 = modéré
3 = important

### Résultats

### 1) Surface de l'érythème

Les résultats sont présentés en terme de surface moyenne (en cm²) de l'érythème induit tous instants confondus, et sont rassemblés dans le tableau 2.

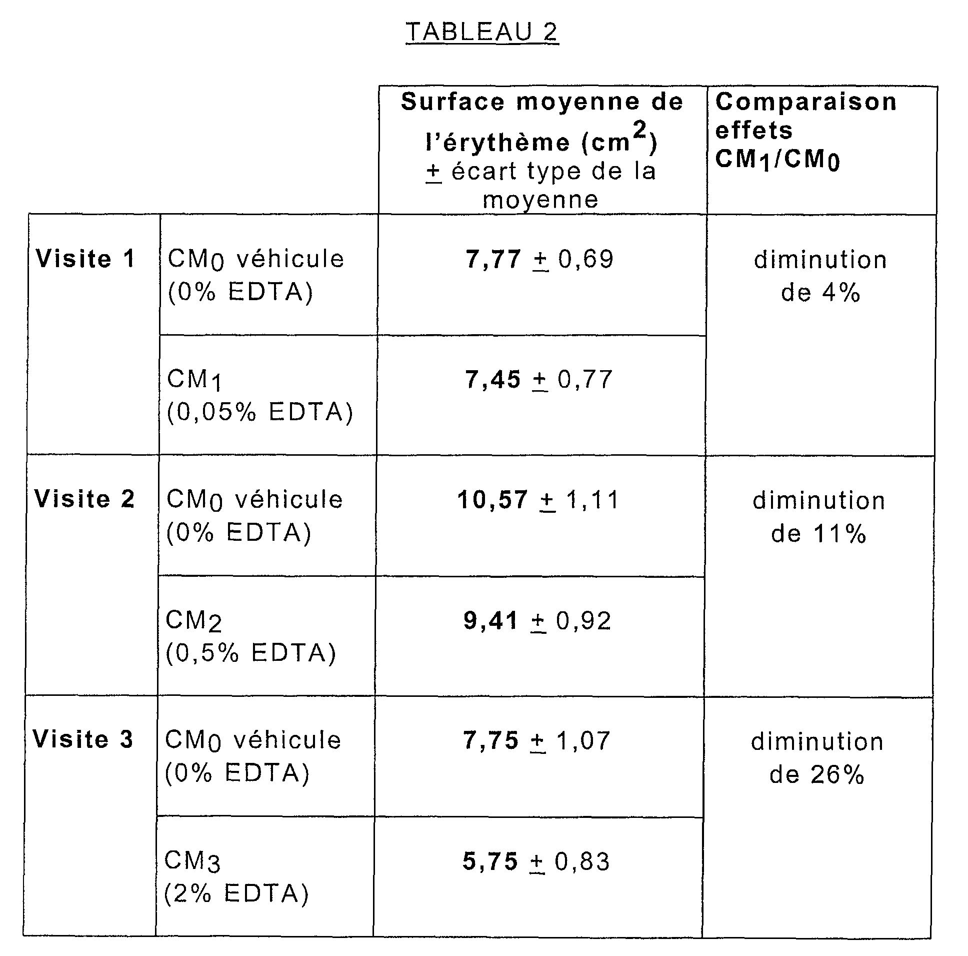

On observe une diminution de la taille de l'érythème induit lorsqu'on applique les compositions CM₁, CM₂ et CM₃, contenant respectivement 0,05%, 0,5% et 2% d'EDTA.

La diminution de la taille de l'érythème est proportionnelle à la concentration d'EDTA.

### 2) Score moyen de prurit

Les résultats sont présentés en terme de score moyen de prurit induit tous instants confondus, et sont rassemblés dans le tableau 3.

On observe une diminution d'environ 50% du prurit pour les compositions CM₁ et CM₂.

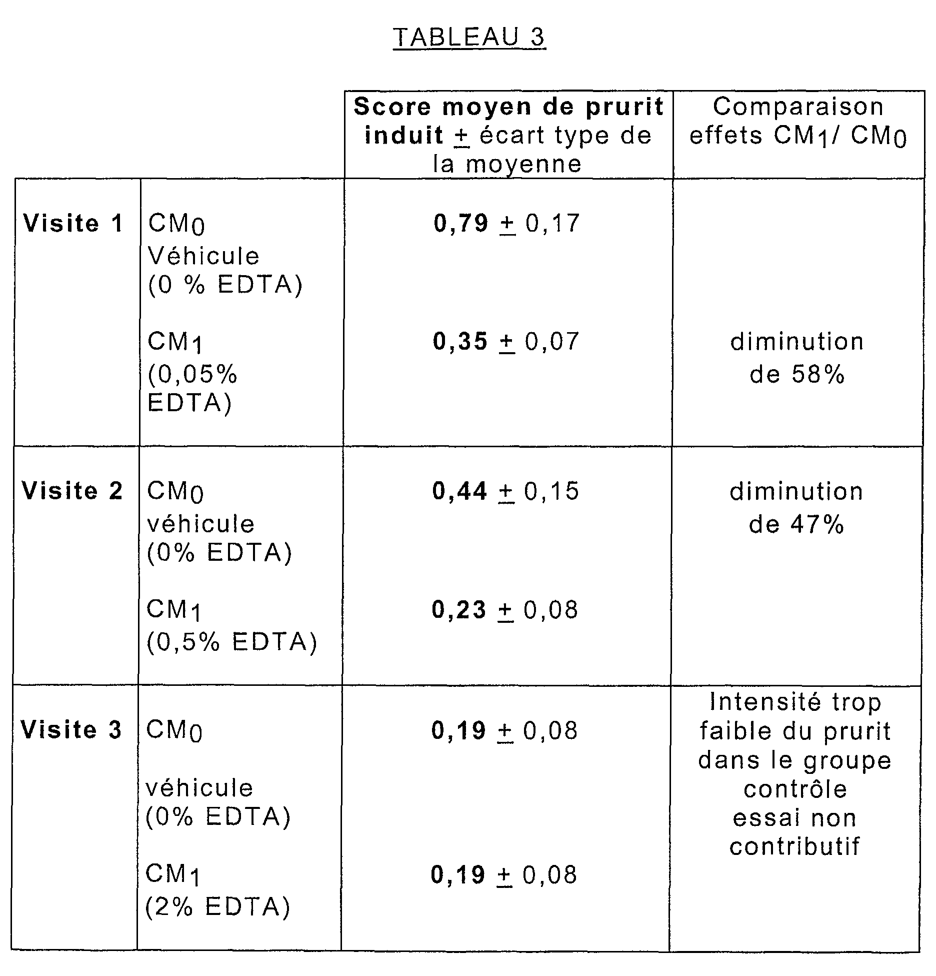

## Revendications

1. Utilisation pour diminuer partiellement ou totalement les symptômes associés à la libération d'histamine dans l'organisme, notamment le prurit et l'érythème, qui comprend l'application topique d'une composition cosmétique et/ou dermatologique et/ou de l'hygiène contenant une quantité effective d'au moins un agent chélateur d'ions, ladite composition étant essentiellement exempte de flavones, de flavonones et/ou de flavonoides et d'agents sequestrants tels que l'acide polyaspartique et ses sels, les dérivés de cellulose et leurs sels, et les copolymères à motifs monomères d'acide maléique ou hydrosuccinique.

2. Utilisation selon la revendication 1, **caractérisée en ce que** l'agent chélateur est un chélateur chimique choisi parmi l'acide éthylènediamine tétracétique (EDTA) et ses sels de sodium, potassium, calcium disodium, de diammonium, de triéthanolamine (TEA-EDTA), l'acide hydroxyéthyl éthylènediamine tétracétique (HEDTA) et son sel trisodique et leurs mélanges.

3. Utilisation selon la revendication 1 ou 2, **caractérisée en ce que** l'agent chélateur est un chélateur biologique choisi parmi la métallothinéine, la transférine, la calmoduline, le chitosan et ses dérivés et leurs mélanges.

4. Utilisation selon l'une quelconque des revendications précédentes, **caractérisée en ce que** l'agent chélateur est présent dans la composition en une quantité représentant de 10⁻⁶ à 10 % en poids, de préférence de 0,1 à 5 % en poids du poids total de la composition.

5. Utilisation selon l'une quelconque des revendications précédentes, **caractérisée en ce que** la composition comprend au moins une phase grasse, liquide ou solide.

6. Utilisation selon la revendication 5, **caractérisée en ce que** le phase grasse est liquide et contient au moins une huile choisie parmi les huiles hydrocarbonées, les huiles végétales, les huiles animales, les huiles de synthèse, les huiles siliconées et les huiles fluorées.

7. Utilisation selon la revendication 5, **caractérisée en ce que** la phase grasse est solide et contient au moins une cire choisie parmi les cires d'origine naturelle telles que les cires d'abeilles, de Carnauba, de paraffine, les esters d'acides gras, les alcools gras et les cires siliconées.

8. Utilisation selon l'une quelconque des revendications précédentes, **caractérisée en ce que** la composition contient au moins un actif cosmétique.

9. Utilisation selon l'une quelconque des revendications précédentes, **caractérisée en ce que** la composition contient au moins un additif choisi parmi l'eau, les antioxydants, les huiles essentielles, les conservateurs, les neutralisants, les polymères liposolubles, les charges, les parfums, les émulsionnants, les gélifiants, les filtres, les absorbeurs d'odeurs et les matières colorantes.

10. Utilisation selon l'une quelconque des revendications précédentes, **caractérisée en ce que** la composition se présente sous forme de solution aqueuse ou huileuse ou de dispersion du type lotion ou sérum, pour une application topique sur la peau.

11. Utilisation selon l'une quelconque des revendications 1 à 9, **caractérisée en ce que** la composition se présente sous forme d'une émulsion de consistance liquide ou semi-liquide du type lait, obtenues par dispersion d'une phase grasse dans une phase aqueuse (H/E) ou d'une phase aqueuse dans une phase grasse (E/H), pour une application topique sur la peau.

12. Utilisation selon l'une quelconque des revendications 1 à 9, **caractérisée en ce que** la composition se présente sous forme de suspension ou d'émulsion de consistance molle du type crème ou gelaqueux ou anhydre, pour une application topique sur la peau.

13. Utilisation selon l'une quelconque des revendications 1 à 9, **caractérisée en ce que** la composition se présente sous forme de solutions aqueuses alcooliques ou hydroalcooliques, pour une application sur le cuir chevelu.

14. Utilisation selon l'une quelconque des revendications 1 à 9, **caractérisée en ce que** la composition se présente sous forme de crèmes, de gels, d'émulsions ou de mousses, pour une application sur le cuir chevelu.

15. Utilisation selon l'une quelconque des revendications 1 à 9, **caractérisée en ce que** la composition est conditionnée sous forme d'alcool et comprend un agent propulseur sous pression, pour une application sur le cuir chevelu.
